# FASCICULE DE BREVET EUROPEEN

(11) **EP 1 013 256 B1**
(45) Date de publication et mention de la délivrance du brevet: **17.10.2001**
(21) Numéro de dépôt: 99403215.9
(22) Date de dépôt: 20.12.1999
(51) Int. Cl.: A61K 7/032, A61K 7/48

(54) **Composition de revêtement des fibres kératiniques**
Zusammensetzung zur Behandlung von Keratinfasern
Composition for treating keratinic fibers

(30) Priorité: 21.12.1998 FR 9816131
(43) Date de publication de la demande: 28.06.2000
(73) Titulaire: L'OREAL, 75008 Paris (FR)
(72) Inventeur: Bodelin, Sophie, 92170 Vanves (FR); Debert, Danièle, 91600 Savigny-Sur-Orge (FR)
(74) Mandataire: Kromer, Christophe

(56) Documents cités:
- EP-A- 0 832 637
- WO-A-96/03964
- FR-A- 2 758 084
- GB-A- 2 238 242

## Description

La présente invention a pour objet une composition de revêtement des fibres kératiniques, notamment des cils et des cheveux d'êtres humains, comprenant un polymère filmogène et une charge. L'invention a également pour objet l'utilisation de cette composition pour recourber les fibres kératiniques, ainsi qu'un procédé de revêtement de ces dernières. La composition et le procédé de revêtement selon l'invention sont plus particulièrement destinés aux fibres kératiniques sensiblement longitudinales telles que les cils, les sourcils et les cheveux, y compris les faux-cils et les postiches. La composition peut être une composition de maquillage, une base de maquillage, une composition à appliquer sur un maquillage, dite encore top-coat, ou bien encore une composition de traitement cosmétique des fibres kératiniques. Plus spécialement, l'invention porte sur un mascara.

Généralement, les compositions de revêtement des cils, appelées mascaras, comprennent des cires pour obtenir un gainage des cils. Ces cires peuvent être dispersées dans un milieu aqueux, notamment à l'aide de tensioactifs. Or le film de maquillage obtenu avec ces compositions a tendance à s'effriter dans le temps. Le film ainsi fragilisé à tendance à ne pas résister aux frottements, notamment des doigts et/ou à l'eau, lors de baignades ou de douches par exemple, ce qui est contraire à l'obtention d'un maquillage présentant une bonne tenue dans le temps.

Il est aussi connu des mascaras résistants à l'eau, dit "waterproof", qui comprennent des cires dans un milieu anhydre, notamment dans une phase grasse liquide comprenant des solvants organiques. Ces compositions peuvent comprendre en outre une phase aqueuse dispersée dans la phase grasse liquide, comme le décrit par exemple le document WO-A-91/12793. Ce document précise par ailleurs que la résistance à l'eau du mascara peut être améliorée en ajoutant dans la phase aqueuse un polymère filmogène hydrosoluble. Toutefois, ces compositions ne permettent pas d'obtenir un bon recourbement des cils.

Il est connu de la demande EP-A-832 637 une composition cosmétique, notamment pour le maquillage des yeux, comprenant une phase aqueuse contenant un polymère filmogène, une phase grasse contenant une cire, et un agent siliconé. La composition peut comprendre également une charge. Lorsque la composition est sous forme de dispersion eau-dans-cire, elle peut contenir un agent épaississant et un solvant organique ou une huile volatile.

La demande WO-A-96/03964 décrit une composition de maquillage de la peau, notamment de fond de teint, contenant des particules de pigments minéraux ou organiques traités en surface avec un polymère dispersible dans l'eau.

Le but de la présente invention est de disposer d'une composition de revêtement des fibres kératiniques, notamment des cils, conduisant, après application, à un revêtement résistant à l'eau et conférant un bon recourbement des fibres kératiniques.

La demanderesse a maintenant constaté de façon surprenante qu'un tel revêtement des fibres kératiniques, notamment des cils, pouvait être obtenu en utilisant un polymère filmogène et une charge lamellaire dans une composition comprenant une phase aqueuse dispersée dans une phase grasse liquide. On obtient alors une composition qui conduit, après application sur les fibres kératiniques, à un revêtement résistant à l'eau et conférant un bon recourbement aux fibres kératiniques. Par ailleurs, le recourbement obtenu est meilleur que celui obtenu avec des charges sphériques comme l'amidon de riz.

De plus, le revêtement ne s'effrite pas et présente une bonne résistance aux frottements, notamment aux frottements des doigts. Le revêtement confère également un effet d'allongement aux cils.

De façon plus précise, la présente invention a pour objet une composition cosmétique de revêtement des fibres kératiniques, notamment des cils, comprenant une phase aqueuse dispersée dans une phase grasse liquide contenant au moins un solvant organique volatil, ladite phase aqueuse contenant un système polymérique comprenant au moins un polymère filmogène, caractérisée par le fait que le polymère filmogène est sous forme de particules solides dispersées dans la phase aqueuse, et que la composition comprend au moins une charge lamellaire.

Un autre objet de l'invention est un produit de mascara comprenant un réservoir contenant une composition de mascara telle que définie précédemment, et muni d'un système d'application de la composition sur les fibres kératiniques, notamment les cils.

L'invention a aussi pour objet un procédé de revêtement des fibres kératiniques, notamment des cils, consistant à appliquer sur les fibres kératiniques une composition telle que définie précédemment.

L'invention a également pour objet l'utilisation d'un système polymérique comprenant au moins un polymère filmogène et au moins une charge lamellaire, dans une composition de revêtement des fibres kératiniques, notamment des cils, pour recourber les fibres kératiniques et/ou résister à l'eau et/ou allonger les fibres kératiniques, ladite composition comprenant une phase aqueuse dispersée dans une phase grasse liquide contenant au moins un solvant organique volatil, ledit polymère filmogène étant sous forme de particules solides dispersées dans ladite phase aqueuse.

Dans la présente demande, on entend par "composition de revêtement des fibres kératiniques" une composition apte à former un film sur les fibres kératiniques.

Par polymère filmogène, on entend un polymère conduisant seul à un film isolable.

Par polymère sous forme de particules solides dispersées dans la phase aqueuse, connu généralement sous le nom de latex ou pseudolatex, on entend une phase aqueuse dans laquelle est dispersé directement le polymère sous forme de particules.

Le polymère filmogène du système polymérique présent sous forme de particules solides dispersées dans la phase aqueuse peut être choisi parmi les polycondensats, les polymères radicalaires, et les polymères d'origine naturelle.

De préférence, ledit système polymérique est apte à former un film produisant à une concentration de 7 % dans l'eau, une rétraction du stratum corneum isolé de plus de 1 % à 30°C sous une humidité relative de 40 %, de préférence de plus de 1,5 %, et mieux de plus de 2 %, pour obtenir un bon recourbement des fibres kératiniques. Cette rétraction est mesurée à l'extensiomètre selon la méthode de mesure indiquée ultérieurement dans les exemples.

Comme polycondensats, on peut citer les polyuréthannes anioniques, cationiques, non ioniques ou amphotères, les polyuréthannes-acryliques, les polyuréthannes-polyvinylpyrrolidones, les polyester-polyuréthannes, les polyéther-polyuréthannes, les polyurée-uréthanes, les polyurées, les polyesters (qui sont décrits notamment dans les brevets US-A-3734874, US-A-4233196, US-A-4304901) et leurs mélanges.

Comme polymère filmogène approprié, on peut utiliser de préférence les polyester-polyuréthanes, et plus particulièrement les polyester-polyuréthanes aptes à former un film ayant une dureté allant de 40 à 200 secondes, et mieux de 50 à 170 secondes. On peut par exemple utiliser les polyester-polyuréthanes vendus sous les dénominations "AVALURE UR-425", "AVALURE UR-430", "AVALURE UR-405", "AVALURE UR-410" par la société GOODRICH.

On peut également utiliser les polyéther-polyuréthanes, et plus particulièrement les polyéther-polyuréthanes aptes à former un film ayant une dureté allant de 10 à 40 secondes, et mieux de 20 à 35 secondes. Comme exemple de polyéther-polyuréthane, on peut citer ceux vendus sous les dénominations "SANCURE 878", "AVALURE UR-450", "SANCURE 861" par la société GOODRICH.

La dureté du film de polymère est mesurée sur un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polymère. La dureté du film est mesurée selon la norme ASTM D-43-66, ou la norme NF-T 30-016 (octobre 1981), à l'aide d'un pendule de Persoz.

Comme polymère filmogène approprié selon l'invention, on peut également utiliser les polyesters anioniques et notamment ceux comprenant au moins un monomère portant un groupement -SO₃M avec M représentant un atome d'hydrogène, un ion ammonium NH₄⁺ ou un ion métallique. Le copolyester peut être par exemple un copolymère d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique.

L'acide dicarboxylique peut être choisi parmi l'acide phtalique, l'acide isophtalique, l'acide téréphtalique. Le diol peut être choisi parmi l'éthylène glycol, le diéthylène glycol, le triéthylène glycol, le 1,3-propanediol, le 1,4-cyclohexane diméthanol, le 1,4-butanediol. Le monomère aromatique bifonctionnel portant le groupement - SO₃M peut être choisi parmi l'acide sulfoisophtalique, notamment le sel de sodium de l'acide 5-sulfo-isophtalique, l'acide sulfotéréphtalique, l'acide sulfophtalique, l'acide 4-sulfonaphtalène-2,7-dicarboxylique.

Comme polyester préféré, on peut utiliser un polyester consistant essentiellement en des unités répétées d'acide isophtalique, de diol et d'acide sulfo-isophtalique, et notament les sulfopolyesters obtenus par condensation de di-éthylèneglycol, cyclohexane di-méthanol, acide isophtalique, acide sulfoisophtalique. Comme sulfopolyester, on peut utiliser ceux commercialisés sous les dénominations AQ55S par la société EASTMAN.

Comme polymères radicalaires, on peut citer les polymères acryliques, les copolymères acrylique/styrène, les copolymères vinyliques comme les copolymères d'esters vinyliques.

Lorsque le polymère filmogène ne permet pas d'obtenir seul un film ayant les caractéristiques mentionnées précédemment, il est possible d'ajouter un composé dont la fonction est de modifier les propriétés du polymère filmogène pour obtenir le système polymérique souhaité. Aussi, selon un mode de réalisation de la composition selon l'invention, ledit système polymérique peut comprendre au moins un agent auxiliaire de filmification permettant d'obtenir un film ayant les caractéristiques telles que décrites précédemment. L'agent auxiliaire de filmification permet notamment d'obtenir un film conférant un bon recourbement aux cils. Dans ce cas, le système polymérique comprend un mélange d'un ou plusieurs polymères filmogènes et d'au moins un agent auxiliaire de filmification.

Un tel agent auxiliaire de filmification peut être choisi parmi tous les composés connus de l'homme du métier comme étant susceptibles de remplir la fonction recherchée, et être notamment choisi parmi les agents plastifiants. En outre, l'agent auxiliaire de filmification peut aussi être choisi parmi les agents de coalescence. Cet agent auxiliaire peut être hydrosoluble ou insoluble dans l'eau et peut éventuellement se présenter sous forme de dispersion aqueuse.
En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

La composition peut comprendre de 0,05 % à 10 % en poids, de préférence de 0,1 % à 7 %, de matière sèche de polymère filmogène sous forme de particules dispersées dans le milieu aqueux, par rapport au poids total de la composition.

Le système polymérique utilisé (polymère(s) ou polymère et plastifiant) selon l'invention peut être notamment présent en une quantité de matière active (M.A.) allant de 0,05 à 15 %, et mieux de 0,1 à 10 % du poids total de la composition.

La taille des particules de polymère filmogène peut aller de 10 nm à 500 nm, et de préférence de 20 nm à 300 nm.

La phase aqueuse de la composition peut être constituée essentiellement d'eau. Elle peut comprendre également un mélange d'eau et de solvant miscible à l'eau comme les monoalcools inférieurs ayant de 1 à 5 atomes de carbone, les cétones en C₃-C₄, les aldéhydes en C₃-C₄. Comme solvant miscible à l'eau, on peut de préférence utiliser de l'éthanol. La teneur en solvant miscible à l'eau peut aller de 0,1 % à 15 % en poids, et mieux de 1 à 8 % en poids, par rapport au poids total de la composition.

Le poids total de la phase aqueuse dans la composition selon l'invention, peut aller de 1 % à 35 % en poids, par rapport au poids total de la composition, et de préférence de 1 % à 20 % en poids.

La phase aqueuse de la composition peut comprendre, en outre, un polymère filmogène additionnel hydrosoluble, notamment présent en une teneur allant de 0,01 à 5 % en poids, par rapport au poids total de la composition.

Comme polymère hydrosoluble, on peut notamment citer :
- les polymères cellulosiques hydrosolubles comme l'hydroxyéthyl cellulose, l'hydroxypropyl cellulose, la carboxyméthyl cellulose, l'hydroxypropyl éthyl cellulose, l'éthyl hydroxyéthyl cellulose
- les dérivés de kératine, tels que les hydrolysats de kératine et les kératines sulfoniques ;
- les dérivés de chitine ou de chitosane anioniques, cationiques, amphotères ou non-ioniques, et notamment l'hydroxy propyl chitosane,
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates;
- les alcools polyvinyliques et les polyvinylpyrrolidones,
- les copolymères vinyliques, tels que les copolymères de l'éther méthylvinylique et de l'anhydride malique, ou le copolymère de l'acétate de vinyle et de l'acide crotonique ;
- les polyéthylèneglycols,
- les polymères d'origine naturelle, éventuellement modifiés, tels que :
   . les gommes arabiques, la gomme de guar, les dérivés du xanthane, la gomme de karaya ;
   . les alginates et les carraghénates ;
   . les glycoaminoglycanes, l'acide hyaluronique et ses dérivés ;
   . la résine shellac, la gomme de sandaraque, les dammars, les élémis, les copals ;
   . l'acide désoxyribonucléïque.

La charge lamellaire présente dans la composition est de préférence une charge minérale lamellaire. Cette charge lamellaire permet d'obtenir, avec le polymère filmogène, un bon recourbement des fibres kératiniques, notamment des cils. La charge lamellaire utilisée selon l'invention peut se présenter sous forme de particules ayant une taille moyenne inférieure à 40 µm, et notamment allant de 0,5 µm à 30 µm.

Comme charge lamellaire, on peut utiliser en particulier :
- le talc qui est un silicate de magnésium hydraté, et notamment ceux commercialisés selon les dénominations "Talc Luzenac 00" par la société LUZENAC, "Talc P3" par la société NIPPON TALC ;
- le kaolin qui est un silicate d'aluminium hydraté qui se présente sous la forme de particules de forme anisotrope ayant des dimensions généralement inférieures à 30 µm ; Comme kaolin, on peut utiliser celui vendu sous la dénomination "Kaolin Suprême 1" de ENGLISH CHINA LAYS.
- le nitrure de bore, et notamment ceux commercialisés sous les dénominations ""Ceram Blanche 1", "Ceram Blanche" par la société SPCI ;
- le mica, ou alumino silicate, qui peut être choisi parmi la muscovite, la phlogopite, la tiotite, la séricite, la lépidolite, la paragonite, la margarite, la roscoelite, le mica artificiel ou synthétique ayant un atome de fluor substituant le groupe hydroxyle du mica naturel, ainsi que les produits cuits ou calcinés de ces micas. Les micas se présentent généralement sous forme d'écailles ayant des dimensions de 2 à 200 µm, de préférence 5-70 µm et une épaisseur de 0,1 à 5 µm, de préférence de 0,2-3 µm. Comme mica, on peut par exemple utiliser ceux vendus sous les dénominations "MICA SFG70" de la société ASPANGER, "MICA CONCORD 1000" par la société SCIAMA ;
- la silice lamellaire, comme notamment celle vendue sous les dénominations "SG Flake 3 M" par la société MAPRECOS, "Chemicelen" par la société SUMITOMO ;
- et leurs mélanges.

On utilise de préférence comme charge lamellaire le talc.

La charge lamellaire peut être présente dans la composition selon l'invention en une teneur allant de 0,5 % à 10 % en poids, par rapport au poids total de la composition, et mieux de 0,5 % à 5 % en poids.

La phase grasse liquide de la composition comprend au moins un solvant organique volatil.

On entend par "phase grasse liquide" dans la présente invention, tout milieu non aqueux liquide à température ambiante et non miscible à l'eau.
On entend par "solvant organique volatil" un solvant organique susceptible de s'évaporer à température ambiante d'un support sur lequel il a été appliqué, autrement dit un solvant ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles facilitent, notamment, l'application de la composition sur les fibres kératiniques. Ces huiles peuvent être des huiles hydrocarbonées ou des huiles siliconées.

Selon un mode de réalisation de l'invention, le solvant organique volatil peut être une huile hydrocarbonée volatile. On entend par "huile hydrocarbonée", une huile ne contenant que des atomes d'hydrogène et de carbone.

Les huiles hydrocarbonées volatiles préférées convenant pour la composition selon l'invention sont en particulier les isoparaffines, à savoir des alcanes ramifiés, comportant de 8 à 16 atomes de carbone telles que les 'ISOPARs', les PERMETYLs et notamment l'isododécane (encore appelé 2,2,4,4,6-pentaméthylheptane). On peut bien entendu également utiliser des mélangés de telles isoparaffines. D'autres huiles hydrocarbonées volatiles comme les distillats de pétrole, notamment ceux vendus sous la dénomination Shell Solt par la société SHELL, peuvent aussi être utilisés.

Le solvant organique volatil peut être présent dans la composition selon l'invention en une teneur allant de 35 % à 75 % en poids, par rapport au poids total de la composition, de préférence de 45 % à 70 % en poids, et mieux de 50 % à 65 % en poids.

Comme solvants organiques volatils on peut utiliser aussi les silicones volatiles, comme par exemple les huiles de silicones cycliques et volatiles telles que l'octaméthylcyclotétrasiloxane, le décaméthylcyclopentasiloxane, le dodécaméthylcyclohexasiloxane, les silicones linéaires volatiles telles que l'octaméthyltrisiloxane, l'heptaméthylhexyltrisiloxane, l'heptaméthyloctyltrisiloxane, ou bien encore les huiles volatiles fluorées telles que le nonafluorométhoxybutane ou le perfluorométhylcyclopentane.

La phase grasse liquide peut également contenir des huiles non volatiles, et notamment des huiles hydrocarbonées et/ou siliconées et/ou fluorées non volatiles.
Comme huile hydrocarbonée non volatile, on peut notament citer :
- les huiles hydrocarbonées d'origine animale telle que le perhydrosqualène;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité;
- les hydrocarbures linéaires ou ramifiés, d'origine minérale ou synthétique tels que la vaseline, les polydécènes, le polyisobutène hydrogéné tel que le parléam;
- les esters et les éthers de synthèse comme les huiles de formule R₁₀COOR₁₁ dans laquelle R₁₀ représente le reste d'un acide gras supérieur comportant de 6 à 29 atomes de carbone et R₁₁ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisonanoate de diéthylène glycol et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol;
- les acides gras supérieurs tels que l'acide myristique, l'acide palmitique, l'acide stéarique, l'acide béhénique, l'acide oléique, l'acide linoléique, l'acide linolénique ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 5 % en poids, par rapport au poids total de la composition, de préférence de 0 % à 2 % en poids, et mieux de 0,1 % à 2 % en poids.

La phase grasse liquide peut contenir en outre des cires pour assurer un bon gainage des cils et maintenir le recourbement.

La cire présente dans la composition selon l'invention peut être choisie parmi les cires d'origine animale, les cires d'origine végétale, les cires d'origine minérale, les cires synthétiques et les fractions diverses de cires d'origine naturelle. Les cires peuvent être présentes en une teneur allant de 2 % à 40 % en poids, par rapport au poids total de la composition, de préférence de 5 % à 30 % en poids, et mieux de 10 % à 25 % en poids.

Avantageusement, la cire peut être choisie parmi les cires (I) ayant un point de fusion allant de 70 °C à 110 °C. Ces cires ont notamment une pénétrabilité à l'aiguille allant de 1 à 7, 5. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Les cires (I) peuvent par exemples être choisies notamment parmi la cire de son de riz, la cire de Carnauba, la cire d'Ouricuri, la cire de Candellila, les cires de Monatan, la cire de canne à sucre, certaines cires de polyéthylène qui répondent aux critères des cires (I).

Avantageusement, la composition selon l'invention peut comprendre une quantité de cires (I) allant de 0,1 % à 20 % en poids, par rapport au poids total de la composition, de préférence de 1 % à 10 % en poids.

Selon un mode de réalisation de la composition selon l'invention, la composition peut comprendre au moins une cire (la) ayant un point de fusion supérieur ou égal à 70 °C et inférieur à 83 °C et/ou une une cire (lb) ayant un point de fusion allant de 83°C à 110°C.

Comme cire (la), on peut par exemple citer la cire de son de riz ou la cire de Candelilla. Comme cire (lb), on peut citer par exemple la cire de Carnauba, la cire d'Ouricuri, les cires de Montan. On utilise de préférence la cire de Carnauba.

Avantageusement, la composition selon l'invention peut comprendre un mélange de cires (I) contenant au moins une première cire (la) et au moins une deuxième cire (lb) telles que définies précédemment.

Ledit mélange de cires (I) peut comprendre de 5 % à 50 % en poids de cire (la), par rapport au'poids total dudit mélange de cires (I), et de 50 % à 95 % en poids de cire (lb).

La composition peut comprendre, en outre, au moins une cire (II), dite cire molle, ayant un point de fusion supérieur ou égal à 45 °C et inférieur à 70 °C. La cire (II) peut avantageusement avoir une pénétrabilité à l'aiguille supérieure à 7,5, et de préférence inférieure ou égale à 217, mesurée selon les conditions définies précédemment pour les cires (I). Cette cire (II) permet notamment d'assouplir le revêtement déposé sur les cils.

Ces cires (II) peuvent être notamment choisies parmi la cire d'abeilles, les cires de lanoline, les cires de paraffine, les cires de cérasine, les cires microcristallines, les ozokérites, les spermaceti, certaines cires de polyéthylène de poids moléculaire tel qu'elles répondent aux critères des cires II, les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en C₈-C₃₂ et qui ont les qualités correspondant à la définition des cires. On peut citer notamment l'huile de tournesol hydrogénée, l'huile de ricin hydrogénée, l'huile de coton hydrogénée, l'huile de coprah hydrogénée et la lanoline hydrogénée.

Avantageusement, la cire (I) et la cire (II) peuvent être présentes dans la composition selon un rapport pondéral cire (I) / cire (II) pouvant aller de 0,2 à 1, et de préférence de 0,4 à 0,7.

La composition selon l'invention peut contenir en outre, dans la phase grasse liquide, un polymère filmogène auxiliaire lipophile. Ce polymère filmogène auxiliaire lipophile peut notamment être soluble, ou dit encore liposoluble, dans la phase grasse liquide. Ce polymère filmogène lipophile confère notamment une bonne tenue de la composition après application sur les fibres kératiniques.

Comme polymère lipophile, on peut notamment coter les copolymères résulatant de la copolyméisation d'au moins un ester vinylique et d'au moins un autre monomère qui peut être une -oléfine, un alkylvinyléther ou un ester allylique ou méthallylique, corne décrits dans la demande FR-A-22622303, dont le contenu est incorporé dans la présente demande à titre de référence.

Comme polymères filmogènes lipophiles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène auxiliaire lipophile de la phase grasse liquide peut être présent dans la composition en une teneur allant de 0,5 % à 15 % en poids, par rapport au poids total de la composition, et mieux de 2 % à 10 % en poids.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX. Ces argiles sont généralement associées, de façon connue, avec un activateur comme le carbonate de propylène ou l'éthanol pour obtenir l'épaississement de la phase grasse liquide.

L'agent épaississant peut être présent en une teneur allant de 0,5 % à 10 % en poids, par rapport au poids total de la composition, et mieux de 1 % à 7 % en poids.

Dans la composition selon l'invention, le poids total de la phase grasse liquide peut aller de 65 % à 99 % en poids, par rapport au poids total de la composition, et mieux de 80 % à 99 % en poids.

La composition peut également comprendre au moins une matière colorante comme les composés pulvérulents et/ou les colorants liposolubles, par exemple à raison de 0,01 à 30 % du poids total de la composition. Les composés pulvérulents peuvent être choisis parmi les pigments et/ou les nacres et/ou les charges, autres que celles décrites précédemment, habituellement utilisés dans les compositions cosmétiques ou dermatologiques. Avantageusement, les composés pulvérulents représentent de 0,1 à 25 % du poids total de la composition'et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition selon l'invention peut également contenir des ingrédients couramment utilisés en cosmétique, tels que les vitamines, les oligo-éléments, les adoucissants, les séquestrants, les parfums, les diméthicone copolyols, les céramides, les agents de cohésion ainsi que les agents alcalinisants ou acidifiants habituellement utilisés dans le domaine cosmétique, les conservateurs, ou leurs mélanges.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée de façon connue pour l'homme du métier en faisant d'abord fondre les cires et en mélangeant les ingrédients de la phase grasse, y compris les additifs lipophiles. On ajoute ensuite les pigments et les charges, ainsi que les solvants préalablement épaissis. On prépare ensuite la phase aqueuse par mélange des constituants et on disperse la phase aqueuse dans la phase grasse.

La composition selon l'invention est destiné à un produit de mascara comprenant un réservoir, contenant ladite composition de mascara, et un système d'application de ladite composition sur les fibres kératiniques, notamment les cils. Le réservoir est muni de façon connue d'une ouverture dans laquelle est logée un système d'essorage. Le système d'application comporte une tige munie à une première extrémité d'une brosse et à une deuxième extrémité d'un bouchon destiné à fermer le réservoir. Un tel conditionnement est notamment illustré à la figure 7 de la demande EP-A-611170 qui est incorporée à titre de référence.

L'invention est illustrée plus en détail dans les exemples suivants.

### Méthode de mesure de rétraction

Le principe consiste à mesurer avant traitement et après traitement la longueur d'une éprouvette de stratum cornéum isolé et de déterminer le pourcentage de rétraction de l'éprouvette.

On utilise des éprouvettes de 1 cm X 0,4 cm de stratum cornéum d'épaisseur allant de 10 à 20 µm disposées sur l'extensiomètre MTT 610 commercialisé par la société DIASTRON.

L'éprouvette est placée entre 2 mâchoires puis laissée pendant 12 heures dans une atmosphère à 30 °C et 40 % d'humidité relative.

On tracte à la vitesse de 2 mm/minute l'éprouvette d'une longueur comprise entre 5 et 10 % de la longueur initiale pour déterminer la longueur I₁ à partir de laquelle l'éprouvette commence à exercer une force sur les mâchoires et détectée par l'appareil.

On détend ensuite l'éprouvette puis on applique sur le stratum cornéum 2 mg d'une composition aqueuse à 7 % en poids de polymère. Après évaporation totale de la composition, on tracte l'éprouvette dans les mêmes conditions que celles décrites précédemment pour déterminer également la longueur I₂.pour l'éprouvette traitée.

Le pourcentage de rétraction est déterminé par le rapport : 100 X (I₂ - I₁)/I₁.

### Exemple 1 :

On a préparé un mascara ayant la composition suivante :
- Cire de carnauba 4,6 g
- Cire de son de riz 2,1 g
- Paraffine 2,2 g
- Cire d'abeille 7,9 g
- Hydrolysat de protéines de blé
   (Tritisol de la société CRODA) 0,34 g
- Talc 1 g
- Bentonite 5 g
- Copolymère acétate de vinyle/stéarate d'allyle (65/35) 6,5 g
- Polylaurate de vinyle (Mexomère PP de CHIMEX) 0,7 g
- Sulfopolyester (AQ 55S d'EASTMAN CHEMICAL) 0,12 g
- Isododécane 53,9 g
- Carbonate de propylène 1,6 g
- D-panthénol 0,2 g
- Pigments 4,9 g
- Conservateurs qs
- Eau qsp 100 g

Après application de ce mascara sur les cils, on obtient un bon recourbement des cils et un maquillage résistant à l'eau.

### Exemple 2 : (comparatif)

On a préparé un mascara ayant la composition suivante, ne faisant pas partie de l'invention:
- Cire de carnauba 4,6 g
- Cire de son de riz 2,1 g
- Paraffine 2,2 g
- Cire d'abeille 7,9 g
- Hydrolysat de protéines de blé
   (Tritisol de la société CRODA) 0,34 g
- Amidon de riz 0,7 g
- Bentonite 5 g
- Copolymère acétate de vinyle/stéarate d'allyle (65/35) 6,5 g
- Polylaurate de vinyle (Mexomère PP de CHIMEX) 0,7 g
- Sulfopolyester (AQ 55S d'EASTMAN CHEMICAL) 0,12 g
- Isododécane 54,2 g
- Carbonate de propylène 1,6 g
- D-panthénol 0,2 g
- Pigments 4,9 g
- Conservateurs qs
- Eau qsp 100 g

On a constaté après application du mascara sur les cils que les cils étaient moins recourbés que ceux maquillés avec la composition de l'exemple 1.

### Exemple 3:

On a préparé un mascara ayant la composition suivante:
- Cire de carnauba 4,7 g
- Cire de candellila 0,5 g
- Paraffine 2 g
- Cire d'abeille 8,2 g
- Kaolin (Kaolin Suprème 1 de ENGLISH CHINA CLAYS) 1,5 g
- Bentonite 5,1 g
- Copolymère acétate de vinyle/stéarate d'allyle (65/35) 6,7 g
- Polylaurate de vinyle (Mexomère PP de CHIMEX) 0,6 g
- Isododécane 57,2 g
- Carbonate de propylène 1,6 g
- Polyuréthane-polyéther en dispersion aqueuse
   38 % de matières sèches (AVALURE UR450 de GOODRICH) 0,76 g
- Acide désoxyribonucléïque 0,02 g
- Pigments 5,2 g
- Conservateurs qs
- Eau qsp 100 g

Après application de ce mascara sur les cils, on obtient un bon recourbement des cils et un maquillage résistant à l'eau.

### Exemple 4 :

On a préparé un mascara ayant la composition suivante :
- Cire de jojoba 1 g
- Cire de carnauba 3,2 g
- Cire de son de riz 3,5 g
- Paraffine 3 g
- Cire d'abeille 7,9 g
- Mica (Mica Concord 1000 de SCIAMA) 2 g
- Bentonite 5 g
- Carbonate de propylène 1,6 g
- Copolymère acétate de vinyle/stéarate d'allyle (65/35) 5,5 g
- Polylaurate de vinyle (Mexomère PP de CHIMEX) 1 g
- Sulfopolyester (AQ 55S d'EASTMAN CHEMICAL) 0,15 g
- Isododécane 55,1 g
- Pigments 5 g
- Conservateurs qs
- Eau qsp 100 g

Après application de ce mascara sur les cils, on obtient un bon recourbement des cils et un maquillage résistant à l'eau.

## Revendications

1. Composition cosmétique de revêtement des fibres kératiniques, notamment des cils, comprenant une phase aqueuse dispersée dans une phase grasse liquide contenant au moins un solvant organique volatil, ladite phase aqueuse contenant un système polymérique comprenant au moins un polymère filmogène, **caractérisée par le fait que** le polymère filmogène est sous forme de particules solides dispersées dans la phase aqueuse, et que la composition comprend au moins une charge lamellaire.

2. Composition cosmétique selon la revendication 1, **caractérisée par le fait que** ledit système polymérique est apte à former un film produisant à une concentration de 7 % dans l'eau, une rétraction du stratum corneum isolé de plus de 1 % à 30°C sous une humidité relative de 40 %.

3. Composition cosmétique selon la revendication 1 ou 2, **caractérisée par le fait que** le polymère filmogène est un polyester anionique.

4. Composition cosmétique selon la revendication 3, **caractérisée par le fait que** le polyester comprend au moins un monomère portant un groupement -SO₃M avec M représentant un atome d'hydrogène, un ion ammonium NH₄⁺ ou un ion métallique.

5. Composition cosmétique selon l'une des revendications 3 ou 4, **caractérisée par le fait que** le polyester est un copolymère d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique.

6. Composition cosmétique selon la revendication 5, **caractérisée par le fait que** l'acide dicarboxylique est choisi dans le groupe formé par l'acide phtalique, l'acide isophtalique, l'acide téréphtalique.

7. Composition cosmétique selon l'une des revendications 5 ou 6, **caractérisée par le fait que** le diol est choisi dans le groupe formé par l'éthylène glycol, le diéthylène glycol, le triéthylène glycol, le 1,3-propanediol, le 1,4-cyclohexane diméthanol, le 1,4-butanediol.

8. Composition cosmétique selon l'une quelconque des revendications 5 à 7, **caractérisée par le fait que** le monomère aromatique bifonctionnel portant le groupement -SO₃M est choisi dans le groupe formé par l'acide sulfoisophtalique, l'acide sulfotéréphtalique, l'acide sulfophtalique, l'acide 4-sulfonaphtalène-2,7-dicarboxylique.

9. Composition cosmétique selon l'une quelconque des revendications 5 à 8, **caractérisée par le fait que** le monomère aromatique bifonctionnel portant le groupement -SO₃M est le sel de sodium de l'acide 5-sulfo-isophtalique.

10. Composition cosmétique selon l'une des revendications 1 ou 2, **caractérisée par le fait que** le polymère filmogène est un polyuréthane anionique.

11. Composition cosmétique selon la revendication précédente, **caractérisée par le fait que** le polymère filmogène est choisi dans le groupe formé par les polyéther-polyuréthanes et les polyester-polyuréthanes.

12. Composition cosmétique selon l'une quelconque des revendications précédentes, **caractérisée par le fait que** le polymère filmogène est présent en une teneur, en matières sèches, allant de 0,05 % à 10 % en poids, par rapport au poids total de la composition, et mieux de 0,1 à 7 % en poids.

13. Composition cosmétique selon l'une quelconque des revendications précédentes, **caractérisée par le fait que** la phase aqueuse comprend en outre au moins un polymère filmogène additionnel hydrosoluble.

14. Composition cosmétique selon l'une quelconque des revendications précédentes, **caractérisée par le fait que** le solvant organique volatil est choisi dans le groupe formé par les huiles hydrocarbonées volatiles.

15. Composition cosmétique selon l'une quelconque des revendications précédentes, **caractérisée par le fait que** le solvant organique volatil est choisi dans le groupe formé par les isoparaffines comportant de 8 à 16 atomes de carbone.

16. Composition cosmétique selon l'une quelconque des revendications précédentes, **caractérisée par le fait que** le solvant organique volatil est présent en une teneur allant de 35 % à 75 % en poids, par rapport au poids total de la composition, de préférence de 45 % à 70 % en poids, et mieux de 50 % à 65 % en poids.

17. Composition cosmétique selon l'une quelconque des revendications précédentes, **caractérisée par le fait que** la phase grasse liquide comprend au moins un polymère filmogène auxiliaire lipophile.

18. Composition cosmétique selon l'une quelconque des revendications précédentes, **caractérisée par le fait qu'**elle comprend en outre au moins une cire.

19. Composition cosmétique selon la revendication 18, **caractérisée par le fait que** la cire est présente en une teneur allant de 2 % à 40 % en poids, par rapport au poids total de la composition, de préférence de 5 % à 30 % en poids, et mieux de 10 % à 25 % en poids.

20. Composition cosmétique selon l'une quelconque des revendications précédentes, **caractérisée par le fait que** la phase grasse liquide comprend au moins une cire (I) ayant un point de fusion allant de 70 °C à 110 °C.

21. Composition cosmétique selon la revendication 20, **caractérisée par le fait que** la cire (I) est présente en une teneur allant de 0,1 % à 20 % en poids, par rapport au poids total de la composition.

22. Composition cosmétique selon l'une quelconque des revendications précédentes, **caractérisée par le fait que** la phase grasse liquide comprend au moins une cire (II) ayant un point de fusion supérieur ou égal à 45 °C et inférieur à 70 °C.

23. Composition cosmétique selon la revendication 22, **caractérisée par le fait que** la cire (II) est présente selon un rapport pondéral cire (1)/cire (II) allant de 0,2 à 1.

24. Composition cosmétique selon l'une quelconque des revendications précédentes, **caractérisée par le fait que** la phase grasse liquide est présente en une teneur allant de 65 % à 99 % en poids, par rapport au poids total de la composition.

25. Composition cosmétique selon l'une quelconque des revendications précédentes, **caractérisée par le fait que** la phase aqueuse est présente en une teneur allant de 1 % à 35 % en poids, par rapport au poids total de la composition.

26. Composition cosmétique selon l'une quelconque des revendications précédentes, **caractérisée par le fait que** la charge lamellaire est choisie dans le groupe formé par le talc, le mica, le nitrure de bore, le kaolin, la silice, ou leurs mélanges.

27. Composition cosmétique selon l'une quelconque des revendications précédentes, **caractérisée par le fait que** la charge lamellaire est présente en une teneur allant de 0,5 % à 10 % en poids, par rapport au poids total de la composition, et mieux de 0,5 % à 5 % en poids.

28. Composition cosmétique selon l'une quelconque des revendications précédentes, **caractérisée par le fait qu'**elle contient en outre au moins un additif choisi dans le groupe formé par les vitamines, les oligo-éléments, les adoucissants, les séquestrants, les parfums, les diméthicone copolyols, les céramides, les agents de cohésion, les conservateurs, ou leurs mélanges.

29. Composition cosmétique selon l'une quelconque des revendications précédentes, **caractérisée par le fait que** la composition est une composition de maquillage, une base de maquillage, une composition à appliquer sur un maquillage, une composition de traitement cosmétique des fibres kératiniques.

30. Produit de mascara comprenant un réservoir, contenant une composition de mascara , et un système d'application de ladite composition sur les fibres kératiniques, **caractérisé par le fait que** la composition est une composition selon l'une quelconque des revendications 1 à 29.

31. Procédé de revêtement des fibres kératiniques, notamment des cils, **caractérisé par le fait que** l'on applique sur les fibres kératiniques une composition selon l'une quelconque des revendications 1 à 29.

32. Utilisation d'un système polymérique comprenant au moins un polymère filmogène et au moins une charge lamellaire, dans une composition de revêtement des fibres kératiniques, notamment des cils, pour recourber les fibres kératiniques et/ou résister à l'eau et/ou allonger les fibres kératiniques, ladite composition comprenant une phase aqueuse dispersée dans une phase grasse liquide contenant au moins un solvant organique volatil, ledit polymère filmogène étant sous forme de particules solides dispersées dans ladite phase aqueuse.

## Claims

1. Cosmetic composition for coating keratin fibres, in particular eyelashes, comprising an aqueous phase dispersed in a liquid fatty phase containing at least one volatile organic solvent, the said aqueous phase containing a polymer system comprising at least one film-forming polymer, **characterized in that** the film-forming polymer is in the form of solid particles dispersed in the aqueous phase, and **in that** the composition comprises at least one lamellar filler.

2. Cosmetic composition according to Claim 1, **characterized in that** the said polymer system is capable of forming a film producing, at a concentration of 7% in water, a retraction of isolated stratum corneum of more than 1% at 30°C under a relative humidity of 40%.

3. Cosmetic composition according to Claim 1 or 2, **characterized in that** the film-forming polymer is an anionic polyester.

4. Cosmetic composition according to Claim 3, **characterized in that** the polyester comprises at least one monomer bearing a group -SO₃M with M representing a hydrogen atom, an ammonium ion NH₄⁺ or a metal ion.

5. Cosmetic composition according to either of Claims 3 and 4, **characterized in that** the polyester is a copolyter of at least one dicarboxylic acid, of at least one diol and of at least one difunctional aromatic monomer bearing a group -SO₃M with M representing a hydrogen atom, an ammonium ion NH₄⁺ or a metal ion.

6. Cosmetic composition according to Claim 5, **characterized in that** the dicarboxylic acid is chosen from the group formed by phthalic acid, isophthalic acid and terephthalic acid.

7. Cosmetic composition according to either of Claims 5 and 6, **characterized in that** the diol is chosen from the group formed by ethylene glycol, diethylene glycol, triethylene glycol, 1,3-propanediol, 1,4-cyclohexanedimethanol and 1,4-butanediol,

8. Cosmetic composition according to any one of Claims 5 to 7, **characterized in that** the difunctional aromatic monomer bearing the group -SO₃M is chosen from the group formed by sulphoisophthalic acid, sulphoterephthalic acid, sulphophthalic acid and 4-sulphonaphthalene-2,7-dicarboxylic acid.

9. Cosmetic composition according to any one of Claims 5 to 8, **characterized in that** the difunctional aromatic monomer bearing the group -SO₃M is the sodium salt of 5-sulphoisophthalic acid.

10. Cosmetic composition according to either of Claims 1 and 2, **characterized in that** the film-forming polymer is an anionic polyurethane.

11. Cosmetic composition according to the preceding claim, **characterized in that** the film-forming polymer is chosen from the group formed by polyether-polyurethanes and polyester-polyurethanes.

12. Cosmetic composition according to any one of the preceding claims, **characterized in that** the film-forming polymer is present in a solids content ranging from 0.05% to 10% by weight relative to the total weight of the composition, and better still from 0.1% to 7% by weight.

13. Cosmetic composition according to any one of the preceding claims, **characterized in that** the aqueous phase also comprises at least one additional water-soluble film-forming polymer.

14. Cosmetic composition according to any one of the preceding claims, **characterized in that** the volatile organic solvent is chosen from the group formed by volatile hydrocarbon-based oils.

15. Cosmetic composition according to any one of the preceding claims, **characterized in that** the volatile organic solvent is chosen from the group formed by isoparaffins containing from 8 to 16 carbon atoms.

16. Cosmetic composition according to any one of the preceding claims, **characterized in that** the volatile organic solvent is present in a content ranging from 35% to 75% by weight relative to the total weight of the composition, preferably from 45% to 70% by weight and better still from 50% to 65% by weight.

17. Cosmetic composition according to any one of the preceding claims, **characterized in that** the liquid fatty phase comprises at least one lipophilic auxiliary film-forming polymer.

18. Cosmetic composition according to any one of the preceding claims, **characterized in that** it also comprises at least one wax.

19. Cosmetic composition according to Claim 18, **characterized in that** the wax is present in a content ranging from 2% to 40% by weight relative to the total weight of the composition, preferably from 5% to 30% by weight and better still from 10% to 25% by weight.

20. Cosmetic composition according to any one of the preceding claims, **characterized in that** the liquid fatty phase comprises at least one wax (I) with a melting point ranging from 70°C to 110°C.

21. Cosmetic composition according to Claim 20, **characterized in that** the wax (I) is present in a content ranging from 0.1% to 20% by weight relative to the total weight of the composition.

22. Cosmetic composition according to any one of the preceding claims, **characterized in that** the liquid fatty phase comprises at least one wax (II) with a melting point of greater than or equal to 45°C and less than 70°C.

23. Cosmetic composition according to Claim 22, **characterized in that** the wax (II) is present in a wax (I)/wax (II) weight ratio ranging from 0.2 to 1.

24. Cosmetic composition according to any one of the preceding claims, **characterized in that** the liquid fatty phase is present in a content ranging from 65% to 99% by weight relative to the total weight of the composition.

25. Cosmetic composition according to any one of the preceding claims, **characterized in that** the aqueous phase is present in a content ranging from 1% to 35% by weight relative to the total weight of the composition.

26. Cosmetic composition according to any one of the preceding claims, **characterized in that** the lamellar filler is chosen from the group formed by talc, mica, boron nitride, kaolin and silica, or mixtures thereof.

27. Cosmetic composition according to any one of the preceding claims, **characterized in that** the lamellar filler is present in a content ranging from 0.5% to 10% by weight relative to the total weight of the composition, and better still from 0.5% to 5% by weight.

28. Cosmetic composition according to any one of the preceding claims, **characterized in that** it also contains at least one additive chosen from the group formed by vitamins, trace elements, softeners, sequestering agents, fragrances, dimethicone copolyols, ceramides, cohesion agents and preserving agents, or mixtures thereof

29. Cosmetic composition according to any one of the preceding claims, **characterized in that** the composition is a make-up composition, a make-up base, a composition to be applied over a make-up or a cosmetic treatment composition for keratin fibres.

30. Mascara product comprising a reservoir, containing a mascara composition, and a system for applying the said composition to keratin fibres, **characterized in that** the composition is a composition according to any one of Claims 1 to 29.

31. Process for coating keratin fibres, in particular eyelashes, **characterized in that** a composition according to any one of Claims 1 to 29 is applied to the keratin fibres.

32. Use of a polymer system comprising at least one film-forming polymer and at least one lamellar filler, in a composition for coating keratin fibres, in particular eyelashes, to curl the keratin fibres and/or to make the keratin fibres water-resistant and/or to lengthen the keratin fibres, the said composition comprising an aqueous phase dispersed in a liquid fatty phase containing at least one volatile organic solvent, the said film-forming polymer being in the form of solid particles dispersed in the said aqueous phase.

## Patentansprüche

1. Kosmetische Zusammensetzung zum Auftragen auf Keratinfasern und insbesondere Wimpern, die eine wäßrige Phase aufweist, die in einer flüssigen Fettphase dispergiert ist, welche mindestens ein flüchtiges organisches Lösungsmittel enthält, wobei die wäßrige Phase ein Polymersystem mit mindestens einem filmbildenden Polymer enthält,
**dadurch gekennzeichnet, daß**
das filmbildende Polymer in Form von festen, in der wäßrigen Phase dispergierten Partikeln vorliegt und dadurch, daß die Zusammensetzung mindestens einen lamellaren Füllstoff enthält.

2. Kosmetische Zusammensetzung nach Anspruch 1, **dadurch gekennzeichnet, daß** das Polymersystem einen Film bilden kann, der in einer Konzentration von 7 % in Wasser bei 30 °C und einer relativen Luftfeuchte von 40 % zu einer Retraktion von isoliertem Stratum corneum von mehr als 1 % führt.

3. Kosmetische Zusammensetzung nach Anspruch 1 oder 2, **dadurch gekennzeichnet, daß** das filmbildende Polymer ein anionischer Polyester ist.

4. Kosmetische Zusammensetzung nach Anspruch 3, **dadurch gekennzeichnet, daß** der Polyester mindestens ein Monomer enthält, das eine Gruppe -SO₃M enthält, wobei M ein Wasserstoffatom, ein Ammoniumion NH₄⁺ oder ein Metallion bedeutet.

5. Kosmetische Zusammensetzung nach einem der Ansprüche 3 oder 4, **dadurch gekennzeichnet, daß** der Polyester ein Copolymer mindestens einer Dicarbonsäure, mindestens eines Diols und mindestens eines bifunktionellen aromatischen Monomers ist, das eine Gruppe -SO₃M trägt, wobei M ein Wasserstoffatom, ein Ammoniumion NH₄⁺ oder ein Metallion bedeutet.

6. Kosmetische Zusammensetzung nach Anspruch 5, **dadurch gekennzeichnet, daß** die Dicarbonsäure unter Phthalsäure, Isophthalsäure und Terephthalsäure ausgewählt ist.

7. Kosmetische Zusammensetzung nach einem der Ansprüche 5 oder 6, **dadurch gekennzeichnet, daß** das Diol unter Ethylenglykol, Diethylenglykol, Triethylenglykol, 1,3-Propandiol, 1,4-Cyclohexandimethanol und 1,4-Butandiol ausgewählt ist.

8. Kosmetische Zusammensetzung nach einem der Ansprüche 5 bis 7, **dadurch gekennzeichnet, daß** das bifunktionelle aromatische Monomer, das die SO₃M-Gruppe trägt, unter Sulfoisophthalsäure, Sulfoterephthalsäure, Sulfophthalsäure und 4-Sulfo-naphthalen-2,7-dicarbonsäure ausgewählt ist.

9. Kosmetische Zusammensetzung nach einem der Ansprüche 5 bis 8, **dadurch gekennzeichnet, daß** das bifunktionelle aromatische Monomer, das die SO₃M-Gruppe trägt, das Natriumsalz von 5-Sulfo-isophthalsäure ist.

10. Kosmetische Zusammensetzung nach einem der Ansprüche 1 oder 2, **dadurch gekennzeichnet, daß** das filmbildende Polymer ein anionisches Polyurethan ist.

11. Kosmetische Zusammensetzung nach dem vorhergehenden Anspruch, **dadurch gekennzeichnet, daß** das filmbildende Polymer unter den Polyether-Polyurethanen und Polyester-Polyurethanen ausgewählt ist.

12. Kosmetische Zusammensetzung nach einem der vorhergehenden Ansprüche, **dadurch gekennzeichnet, daß** das filmbildende Polymer in einem Trockensubstanzgehalt von 0,05 bis 10 Gew.-%, bezogen auf das Gesamtgewicht der Zusammensetzung, und besser 0,1 bis 7 Gew.-% vorliegt.

13. Kosmetische Zusammensetzung nach einem der vorhergehenden Ansprüche, **dadurch gekennzeichnet, daß** die wäßrige Phase ferner mindestens ein zusätzliches wasserlösliches filmbildendes Polymer enthält.

14. Kosmetische Zusammensetzung nach einem der vorhergehen den Ansprüche, **dadurch gekennzeichnet, daß** das flüchtige organische Lösungsmittel unter den flüchtigen Kohlenwasserstoffölen ausgewählt ist.

15. Kosmetische Zusammensetzung nach einem der vorhergehenden Ansprüche, **dadurch gekennzeichnet, daß** das flüchtige organische Lösungsmittel unter den Isoparaffinen mit 8 bis 16 Kohlenstoffatomen ausgewählt ist.

16. Kosmetische Zusammensetzung nach einem der vorhergehenden Ansprüche, **dadurch gekennzeichnet, daß** das flüchtige organische Lösungsmittel in einem Mengenanteil von 35 bis 75 Gew.-%, bezogen auf das Gesamtgewicht der Zusammensetzung, vorzugsweise 45 bis 70 Gew.-% und besser 50 bis 65 Gew.-% vorliegt.

17. Kosmetische Zusammensetzung nach einern der vorhergehenden Ansprüche, **dadurch gekennzeichnet, daß** die flüssige Fettphase mindestens ein zusätzliches lipophiles filmbildendes Polymer enthält.

18. Kosmetische Zusammensetzung nach einem der vorhergehenden Ansprüche, **dadurch gekennzeichnet, daß** sie ferner mindestens ein Wachs enthält.

19. Kosmetische Zusammensetzung nach Anspruch 18, **dadurch gekennzeichnet, daß** das Wachs in einem Mengenanteil von 2 bis 40 Gew.-%, bezogen auf das Gesamtgewicht der Zusammensetzung, vorzugsweise 5 bis 30 Gew.-% und besser 10 bis 25 Gew.-% vorliegt.

20. Kosmetische Zusammensetzung nach einem der vorhergehenden Ansprüche, **dadurch gekennzeichnet, daß** die flüssige Fettphase mindestens ein Wachs (I) mit einem Schmelzpunkt von 70 bis 110 °C enthält.

21. Kosmetische Zusammensetzung nach Anspruch 20, **dadurch gekennzeichnet, daß** das Wachs (I) in einer Menge von 0,1 bis 20 Gew.-%, bezogen auf das Gesamtgewicht der Zusammensetzung, vorliegt.

22. Kosmetische Zusammensetzung nach einem der vorhergehenden Ansprüche, **dadurch gekennzeichnet, daß** die flüssige Fettphase mindestens ein Wachs (II) mit einem Schmelzpunkt von 45 °C oder darüber und unter 70 °C aufweist.

23. Kosmetische Zusammensetzung nach Anspruch 22, **dadurch gekennzeichnet, daß** das Wachs (II) in einem Gewichtsverhältnis von Wachs (I)/Wachs (II) von 0,2 bis 1 vorliegt.

24. Kosmetische Zusammensetzung nach einem der vorhergehenden Ansprüche, **dadurch gekennzeichnet, daß** die flüssige Fettphase in einer Menge von 65 bis 99 Gew.-%, bezogen auf das Gesamtgewicht der Zusammensetzung, vorliegt.

25. Kosmetische Zusammensetzung nach einem der vorhergehenden Ansprüche, **dadurch gekennzeichnet, daß** die wäßrige Phase in einer Menge von 1 bis 35 Gew.-%, bezogen auf das Gesamtgewicht der Zusammensetzung, vorliegt.

26. Kosmetische Zusammensetzung nach einem der vorhergehenden Ansprüche, **dadurch gekennzeichnet, daß** der lamellare Füllstoff unter Talk, Glimmer, Bornitrid, Kaolin, Kieselsäure oder deren Gemischen ausgewählt ist.

27. Kosmetische Zusammensetzung nach einem der vorhergehenden Ansprüche, **dadurch gekennzeichnet, daß** der lamellare Füllstoff in einer Menge von 0,5 bis 10 Gew.-%, bezogen auf das Gesamtgewicht der Zusammensetzung, und besser 0,5 bis 5 Gew.-% vorliegt.

28. Kosmetische Zusammensetzung nach einem der vorhergehenden Ansprüche, **dadurch gekennzeichnet, daß** sie ferner mindestens einen Zusatzstoff enthält, der unter den Vitaminen, Spurenelementen, reizlindernden Mitteln, Maskierungsmitteln, Parfums, Dimethiconcopolyolen, Ceramiden, Kohäsionsmitteln, Konservierungsmitteln oder deren Gemischen ausgewählt ist.

29. Kosmetische Zusammensetzung nach einem der vorhergehenden Ansprüche, **dadurch gekennzeichnet, daß** es sich bei der Zusammensetzung um eine Zusammensetzung zum Schminken, eine Grundmasse zum Schminken, eine Zusammensetzung, die auf eine Schminke aufgetragen wird, oder eine Zusammensetzung zur kosmetischen Behandlung von Keratinfasern handelt.

30. Mascaraprodukt, das einen Behälter, der eine Mascarazusammensetzung enthält, und ein System zum Auftragen der Zusammensetzung auf die Keratinfasern umfaßt, **dadurch gekennzeichnet, daß** die Zusammensetzung eine Zusammensetzung nach einem der Ansprüche 1 bis 29 ist.

31. Verfahren zum Überziehen von Keradnfasern und insbesondere Wimpern, **dadurch gekennzeichnet, daß** auf die Keratinfasern eine Zusammensetzung nach einem der Ansprüche 1 bis 29 aufgebracht wird.

32. Verwendung eines Polymersystems, das mindestens ein filmbildendes Polymer und mindestens einen lamellaren Füllstoff enthält, in einer Zusammensetzung zum Auftragen auf Keratinfasern und insbesondere Wimpern, um die Keratinfasern zu krümmen und/oder wasserbeständig zu machen und/oder die Keratinfasern zu verlängern, wobei die Zusammensetzung eine wäßrige Phase aufweist, die in einer flüssigen Fettphase dispergiert ist, die mindestens ein flüchtiges organisches Lösungsmittel enthält, wobei das filmbildende Polymer in Form von festen, in der wäßrigen Phase dispergierten Partikeln vorliegt.
